(19) **Europäisches Patentamt**

**European Patent Office**

**Office européen des brevets**

(11) Veröffentlichungsnummer: **0 338 380 B1**

(12) # EUROPÄISCHE PATENTSCHRIFT

(45) Veröffentlichungstag der Patentschrift: **28.10.92**

(21) Anmeldenummer: **89106356.2**

(22) Anmeldetag: **11.04.89**

(51) Int. Cl.⁵: **A61M 1/00**, A61M 25/00

(54) **Absaugkatheter zum Einführen in die Luftröhre und das Bronchialsystem.**

(30) Priorität: **16.04.88 DE 3812754**

(43) Veröffentlichungstag der Anmeldung:
**25.10.89 Patentblatt 89/43**

(45) Bekanntmachung des Hinweises auf die
Patenterteilung:
**28.10.92 Patentblatt 92/44**

(84) Benannte Vertragsstaaten:
**AT BE CH DE ES FR GB GR IT LI LU NL SE**

(56) Entgegenhaltungen:
**DE-B- 2 364 119**
**DE-C- 3 608 943**
**DE-U- 7 035 135**
**DE-U- 8 310 315**
**US-A- 4 716 896**

**Prospeckt: "Profil Cath", Firma Willy Rüsch AG**

**Prospekt Métras-Katheter, Kat. Nr. 1645-1651**

(73) Patentinhaber: **Schön, Rudolf**
**Am Kümpel 18**
**W-5300 Bonn 1(DE)**

Patentinhaber: **Schmidt, Christoph, Dr.**
**Ferdinandstrasse 10**
**W-5300 Bonn 1(DE)**

Patentinhaber: **Russ, Jürgen**
**Rosental 32**
**W-5300 Bonn 1(DE)**

(72) Erfinder: **Schön, Rudolf**
**Am Kümpel 18**
**W-5300 Bonn 1(DE)**
Erfinder: **Schmidt, Christoph, Dr.**
**Ferdinandstrasse 10**
**W-5300 Bonn 1(DE)**
Erfinder: **Russ, Jürgen**
**Rosental 32**
**W-5300 Bonn 1(DE)**

(74) Vertreter: **Müller-Gerbes, Margot**
**Friedrich-Breuer-Strasse 112**
**W-5300 Bonn 3 (Beuel)(DE)**

## Beschreibung

Die Erfindung betrifft einen rohrförmigen biegsamen Absaugkatheter zum Einführen in die Luftröhre und das Bronchialsystem, enthaltend mindestens ein vom proximalen Ende bis zum distalen Ende des Absaugkatheters durchgängiges Lumen zum Absaugen von Flüssigkeiten aus der Lunge.

Absaugkatheter der gattungsgemäßen Art sind aus der DE-AS 23 64 119 mit einem Lumen zum Absaugen und aus der DE-PS 36 08 943 mit zwei durchgängigen Lumen bekannt, von denen das erste zum Absaugen und das zweite zum Durchleiten von Medikamenten dient. Katheter für die simultane Beatmung und Sauerstoffzufuhr, die in die Luftröhre eingeführt werden, sind beispielsweise aus der US-PS 43 00 550 bekannt. Ein endotrachealer Beatmungstubus, in den ein Absaugdauerkatheter eingesetzt ist, ist aus der DE-OS 23 08 400 bekannt geworden.

Um das Einführen eines Endotrachealtubus in den Kehlkopf und die obere Luftröhre ohne Irritationen und möglichst schmerzfrei zu ermöglichen, ist gemäß DE-GM 70 35 135 bereits vorgeschlagen worden, das distale Ende eines Endotrachealtubus mit einer ersten Krümmung und dessen proximales Ende mit einer zweiten Krümmung zu versehen, um den Tubus etwa S-förmig der Krümmung des Respirationstraktes des Patienten nachzubilden. Einen derartigen Endotrachealtubus kann man jedoch nicht bis in die Bronchien einführen, um direkt an der Lunge abzusaugen.

Auch aus der DE-AS 1211 360 ist ein Bronchialkatheter mit einem rohrförmigen äußeren Führungsorgan aus starrem Werkstoff bekannt geworden, dessen distales Ende um annähernd 90° in Anpassung an den Respirationstrakt abgebogen ist, um leichter am Kehlkopf vorbeigeführt zu werden.

Das Absaugen des Luftröhren- und Bronchienraumes muß sowohl über den rechten als auch den linken Lungenflügel erfolgen. Aufgrund der etwas unsymmetrischen Ausbildung der Bifurkation ergibt sich jedoch in der Praxis, daß die üblicherweise gerade und rohrförmig ausgebildeten biegsamen Absaugkatheter beim normalen Einführen durch die Luftröhre in der Regel zu 70 % in den rechten Lungenflügel eingeführt werden und höchstens zu 30 % auch der linke Lungenflügel erreicht wird. Um diesem Übelstand abzuhelfen, sind bereits Absaugkatheter bekannt geworden, die am Ende zweifach, jedoch jedesmal um 90° gegeneinander versetzt in der Weise abgebogen sind, so daß sie einen dreidimensionalen Haken bilden, jedoch ist für jeden Lungenflügel ein separater in den entsprechenden zwei verschiedenen Richtungen und Ebenen gebogener Absaugkatheter erforderlich. Das Prospekt für den Métras-Katheter mit den Katalog-Nummern 1645-1651 zeigt ebenfalls ein Paar in zwei verschiedenen Richtungen und Ebenen gebogener Absaugkatheter. Schließlich ist aus der US-A-4 716 896 ein speziell für den linken Bronchialast geeigneter Katheter bekannt geworden. Das bedeutet jeweils einen erheblichen Aufwand bei der Behandlung, da für die rechtsseitige Absaugung der Lunge und für die linksseitige Absaugung der Lunge jeweils ein gesonderter Absaugkatheter einzuführen ist, wobei diese Absaugkatheter nicht verwechselt werden dürfen.

Der Erfindung liegt die Aufgabe zugrunde, einen rohrförmigen biegsamen Absaugkatheter für das Bronchialsystem so auszubilden, daß derselbe Absaugkatheter einwandfrei und mit an Sicherheit grenzender Wahrscheinlichkeit in vorbestimmter Weise sowohl in den rechten Lungenflügel oder in den linken Lungenflügel eingeführt werden kann.

Diese Aufgabe wird erfindungsgemäß dadurch gelöst, daß der Bereich des distalen Endes des Absaugkatheters in Längserstreckung des Absaugkatheters wellenförmig ausgebildet ist, und zwar etwa W-förmig gewellt ist und sich diese Wellung in nur einer Ebene erstreckt. Der distale Endbereich des Absaugkatheters ist also nur in einer Ebene gewellt, so daß er nunmehr nach Art einer Feder beim Einführen in die Luftröhre oder Durchführen durch einen Beatmungstubus an den Wänden der Luftröhre bzw. des Beatmungstubus auf einer Seite mit einem Wellenberg und auf der anderen Seite mit dem Ende zum Anliegen kommt und auf diese Weise das distale Ende mit dem Lumenausgang gezielt in eine der beiden Gabelungen der Lungenflügel an der Bifurkation vorbeigeführt werden kann. Je nachdem in welche Richtung das distale gewellte Ende des Absaugkatheters zeigt, wird dieses zwangsläufig in den rechten oder den linken Lungenflügel eingeführt. Die Vorgabe, ob der Absaugkatheter in den rechten Lungenflügel oder in den linken Lungenflügel eingeführt werden soll, ist durch die Haltung des Absaugkatheters bzw. Drehen um 180° auslösbar.

Bei der etwa W-förmigen Wellung des distalen Endes können der erste Wellenbauch und der freie Schenkel des "W" die beiden einander diametral gegenüberliegenden Auflagepunkte oder Anlagepunkte des Absaugkatheters in der Luftröhre bilden, wodurch die definierte Führung des Absaugkatheters in die Lungenflügel ermöglicht wird. Die biegsamen Absaugkatheter aus Kunststoff oder Naturkautschuk oder Weichgummi dürfen jedoch nicht zu weich sein, da sie dann keine ausreichende Stabilität mehr aufweisen und leicht abknicken könnten bzw. zum Verdrehen neigen. Die W-förmige Wellung ist als dauerhafte bleibende Verformung des distalen Endbereiches des flexiblen Absaugkatheters ausgebildet.

Die in die Bronchien durch die Luftröhre einzuführenden Absaugkatheter weisen in der Regel eine Länge von etwa 30 bis 55 cm auf. Zweckmäßigerweise ist erfindungsgemäß vorgesehen, daß der distale Bereich bis zum Ende des Absaugkatheters auf einer Länge von etwa 40 bis 80 mm wellenförmig ausgebildet ist. Damit wird auch der Gabelungsbereich an der Bifurkation einwandfrei überbrückt und die Einführung des distalen Ende des Absaugkatheters in den gewünschten Lungenflügel gefördert.

In weiterer Ausbildung der Erfindung ist vorgesehen, daß die Wellung zum distalen Ende hin gedämpft ist. Dies bedeutet, daß der erste aus der Längsachse des Absaugkatheters herausgeführte Wellenberg die größte Amplitude hat und die in Richtung des distalen Endes nächstfolgenden Wel-lenberge jeweils abnehmende Amplituden aufwei-sen. Eine ausreichende Führung des distalen Endes und des Absaugkatheters wird bereits durch Wellen des Endbereiches über mindestens eine bis zu etwa zwei Wellenlängen erzielt. Um eine Verbesserung der Federwirkung und damit der Anlegewirkung und Führung des distalen Endes des Absaugkatheters beim Einführen durch die Luftröhre oder einen Beatmungstubus zu erzielen, ist des weiteren vorgesehen, daß die Wellenlängsachse gegenüber der Längsachse des Absaugkatheters leicht geneigt ist. Diese Neigung kann einen Winkel $\beta$ von etwa 10 bis 40 Grad, vorzugsweise 15 bis 35 Grad, aufweisen.

Entsprechend der Geometrie der Luftröhre und Bronchien weisen Absaugkatheter in der Regel einen mittleren Außendurchmesser von etwa 3 bis 10 mm auf bei einem Durchmesser des Lumens von etwa 2 bis 8 mm. Zweckmäßigerweise ist nun vorgesehen, daß die Größe der maximalen Amplitude des ersten Wellenberges etwa der Größe des mittleren Außendurchmessers des Absaugkatheters entspricht. Bei sehr kleinem Außendurchmesser des Absaugkatheters kann die Amplitude jedoch auch größer als dieser sein; ebenso bei Ausbildung von nur etwa ein und einer halben Welle am distalen Ende des Absaugkatheters. Die Wellenausschläge des Endes aus der Längsachse des Absaugkatheters nach beiden Richtungen sollen insgesamt ein Maß nicht überschreiten, das etwa einem zwei- bis dreifachen mittleren Außendurchmesser des Absaugkatheters entspricht. Ein solcher Absaugkatheter ist auch noch durch einen Beatmungstubus in die Bronchien einführbar.

In weiterer Ausgestaltung des erfindungsgemäßen Absaugkatheters ist vorgesehen, daß das gewellte distale Ende mit einem aus der Längsachse des Absaugkatheters unter einem spitzen Winkel a, der etwa 10 bis 45 Grad betragen kann, ausgelenkten Wellenschenkel endet.

Um Schleimhautverletzungen zu vermeiden, kann der Absaugkatheter in bekannter Weise am distalen Ende außenseitig mit einer Ringwulst umgeben sein. Ebenso können im Bereich des distalen Endes über den Umfang verteilt ein oder meh-rere Radialöffnungen vorgesehen sein. Üblicherwei-se verläuft der Ausgang der Lumen und der Endquerschnitt des distalen Endes etwa senkrecht zur Längsachse des Absaugkatheters. Durch die erfin-dungsgemäße wellenförmige Ausbildung des En-des verläuft diese aufgrund der Abbiegung nicht mehr senkrecht zur Längsachse des Absaugkatheters. Wenn der Absaugkatheter nicht nur zum Absaugen benutzt werden soll, sondern beispielsweise bei doppellumiger Ausbildung auch zum Einführen von Medikamenten, so kann es zweckmäßig sein, den erfindungsgemäßen einlumigen oder doppellumigen Absaugkatheter so auszugestalten, daß der Endquerschnitt des distalen Endes senkrecht zur Längsachse des Wellenschenkels verläuft, also praktisch hinterschnitten ausgebildet ist. Damit ist der Austritt von Medikamenten in Strahlform in Richtung der Längsachse des Absaugkatheters in die Lungenkanäle gewährleistet. Das distale Ende des Absaugkatheters kann auch abgerundet ausgebildet werden.

Der erfindungsgemäße Absaugkatheter ist sowohl für das Einführen in den rechtsseitigen als auch in den linksseitigen Lungenflügel geeignet. Hierbei wird so vorgegangen, daß nach dem Einführen des Absaugkatheters in den z.B. rechten Lungenflügel dieser anschließend wieder teilweise bis in die Luftröhre zurückgezogen wird, dann um die Längsachse um 180 Grad gedreht und dann erneut wieder eingeschoben wird, wobei er jetzt in den linken Lungenflügel gelangt. Es ist vorteilhaft, den Absaugkatheter auf seiner ganzen Länge mit Längenmaßen zu markieren. Auf diese Weise kann sichergestellt werden, daß das bei einer Behand-lung notwendige Herausziehen des Absaugkathe-ters aus einem Lungenflügel um ihn nach dem Drehen wieder in den anderen Lungenflügel einzuführen, in dem notwendigen Maß erfolgen kann, also weder zuviel noch zuwenig. Darüber hinaus ist es möglich, den Absaugkatheter mit einem Röntgenkonstraststreifen oder dergleichen, der sich über die ganze Länge des Absaugkatheters erstreckt, auszurüsten.

Um das Anhaften des Absaugkatheters an den Wänden der Luftröhre oder eines Beatmungstubus zu vermeiden, kann der Absaugkatheter eine glatte Außenhaut und ggf. noch eine zusätzliche Gleitbeschichtung, z.B. auf Silikonbasis, aufweisen.

Nach einem weiteren Vorschlag der Erfindung ist der Absaugkatheter außenseitig fein gerippt, d.h. mit in Längserstreckung des Absaugkatheters verlaufenden Rippen ausgestattet. Hierbei sind viele kleine Rippen, zwischen denen kleine Rillen verlaufen, bevorzugt. Es sind mindestens vier, vorzugsweise sechs oder mehr Rippen mit einer Höhe von etwa 30 bis 700 $\mu$ gleichmaßig uber den Außenum-

fang des Absaugkatheters verteilt vorgesehen. Auf diese Weise wird erfindungsgemäß die Anlagefläche des Absaugkatheters an der Lufröhre bzw. an der Innenseite des Beatmungstubus beim Einführen verringert, damit wird die Reibungsfläche kleiner und der Absaugkatheter gleitet leichter und besser, so daß er beim Einschieben in die Luftröhre bzw. den Beatmungstubus nicht torquiert (sich nicht verdreht).

Die erfindungsgemäße Ausstattung eignet sich in der Anwendung für alle Arten von Absaugkathetern, die zur Behandlung des Bronchialsystems in die Luftröhre eingeführt werden, sowohl durch die Nase, Luftröhre als auch durch die Trachea, mit und ohne Beatmungstubus. Für die Rechts- oder Linkshaltung des erfindungsgemäß ausgestatteten Absaugkatheters zum Einführen in den rechten oder linken Lungenflügel kann eine entsprechende Markierung am proximalen Ende des Absaugkatheters vorgesehen sein.

Der erfindungsgemäße Absaugkatheter kann durch Extrusion eines rohrförmigen Kunststoffhohlprofiles mit außenseitigen Rippen hergestellt werden. Dieses Kunststoffhohlprofil wird gereckt und dann in vorgegebenen Längen abgelängt oder umgekehrt. Bei diesen einem Absaugkatheter entsprechenden rohrförmigen biegsamen Abschnitten muß dann der spätere distale Endbereich noch gewellt werden. Hierzu wird vorgeschlagen, einen die gewünschte Wellung aufweisenden Stützdraht vorzuformen und diesen vorgeformten Stützdraht in das eine Ende des rohrförmigen Abschnittes einzuführen, wobei der rohrförmige Abschnitt eng an dem Stützdraht anliegt. Danach wird der so vorgeformte gewellte Abschnitt mit dem Stützdraht auf Verformungstemperatur des Kunststoffes erwärmt, je nach Kunststoff zwischen etwa 65 bis 120°C und danach direkt in einem kalten Wasserbad abgeschreckt. Dann kann der Stützdraht entfernt werden. Damit der Stützdraht nicht an dem Abschnitt anklebt, kann er mit einer Antihaftschicht, z.B. Teflon, beschichtet sein. Es ist auch möglich, den rohrförmigen biegsamen Abschnitt in eine zweiteilige Form einzulegen, die der Außenkontur des Absaugkatheters mit gewelltem distalem Ende entspricht, und die Form mit dem eingelegten Abschnitt auf eine ausreichende Verformungstemperatur für den Kunststoff zu erwärmen und danach den vorgeformten Abschnitt abzuschrecken. In jedem Falle wird eine bleibende Verformung des distalen Endbereiches des Absaugkatheters in der gewünschten gewellten Form erreicht, die gleichzeitig eine gewisse Versteifung des Katheterendes bewirkt, wodurch das Einführen des Absaugkatheters in die Bronchien erleichtert wird.

Die Erfindung wird in der Zeichnung an Ausführungsbeispielen erläutert. Es zeigen

Figur 1 einen einlumigen Absaugkatheter in Aufsicht

Figur 2 eine Prinzipdarstellung der Ausbildung des gewellten distalen Endbereiches

Figur 3 und 4 die schematische Darstellung des Einführens des Absaugkatheters in die Lungenflügel

Figur 5 einen zweilumigen Absaugkatheter in Aufsicht

Figur 6 Absaugkatheter mit verschiedenen distalen Enden in Teilansicht

Figur 7 Aufsicht auf einen gerippten Absaugkatheter

Figur 8 Querschnitt DD von Figur 7.

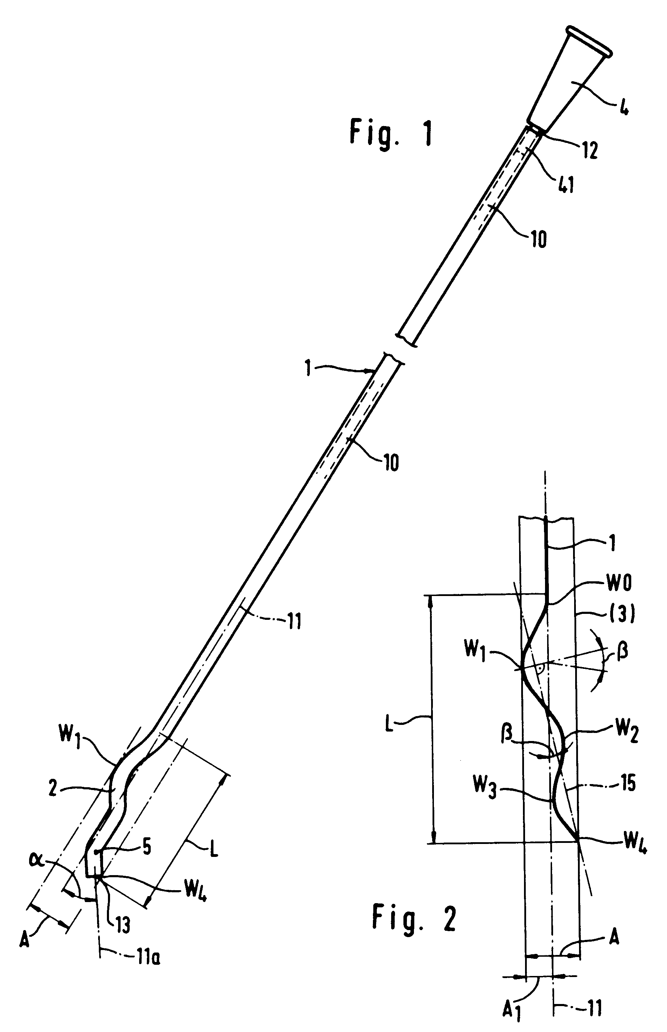

Der Absaugkatheter nach Figur 1 mit einer Länge von etwa 50 cm ist aus einem biegsamen transparenten thermoplastischen Kunststoff durch Extrusion mit nachfolgendem Recken und plastischer bleibender Verformung hergestellt. Der Absaugkatheter 1 hat ein vom proximalen Ende 12 zum distalen Ende 13 mit Lungenausgang durchgehendes Lumen 10 zum Absaugen. Der Durchmesser des Lumens 10 beträgt etwa vier Millimeter bei einem Außendurchmesser von etwa 6 bis 7 mm. Am proximalen Ende 12 ist der Trichter 4 mit dem Stutzen 41 in den Lumeneingang eingeschoben. Es ist auch möglich, den Trichter 4 direkt am proximalen Ende 12 des Absaugkatheters anzuformen, wodurch dieser ein konisch erweitertes Ende erhält. Im Bereich des distalen Endes 13 sind radiale Öffnungen über den Umfang verteilt in dem Absaugkatheter vorgesehen, die bis zum Lumen durchgehen, mindestens eine Öffnung ist ausgebildete. Das distale Ende kann außenseitig mit der Ringwulst 6 umgeben sein. Der Absaugkatheter 1 hat die Längsachse 11. Der distale Bereich 2 ist zum Ende 13 hin über die Länge L gewellt ausgebildet. Die Wellung erfolgt in einer Ebene und hat etwa W-förmige Gestalt, wobei die Wellung gedämpft zum distalen Ende 13 hin verläuft, d.h. mit abnehmender Amplitude der Wellenberge, siehe auch Figur 2. Das in Figur 1 dargestellte Beispiel hat etwa Originalgröße. Die Wellenlängsachse 15 ist gegenüber der Längsachse 11 des Absaugkatheters 1 unter dem Winkel $\beta$ leicht geneigt. Dies wird beispielsweise dadurch möglich, daß der Absaugkatheter zu Beginn der Wellung schwach mit einem sehr kleinen Wellental W0 aus der Längsachse 11 ausgelenkt wird und dann in die gewünschte W-Form mit der ersten großen Welle mit der maximalen Amplitude überführt wird. Die Wellung des distalen Endbereiches soll so erfolgen, daß nach jeder Seite in bezug auf die Längsachse 11 des Absaugkatheters ein Anlagepunkt an der Luftröhre bzw. dem Beatmungstubus erzielt werden kann, wodurch der Absaugkatheter abgestützt eingeführt wird, so daß er nicht abknicken kann. Bei dem gezeigten Beispiel nach Figur 1 und 2 sind das die Anlagepunkte W1 am ersten maximalen Wellenberg und auf der gegenüberliegenden Seite der Anlagepunkt W4, d.h. das Ende des freien Schenkels der letzten Welle am distalen Ende. Die Luftröhre ist in Figur 2 schematisch mit 3 markiert.

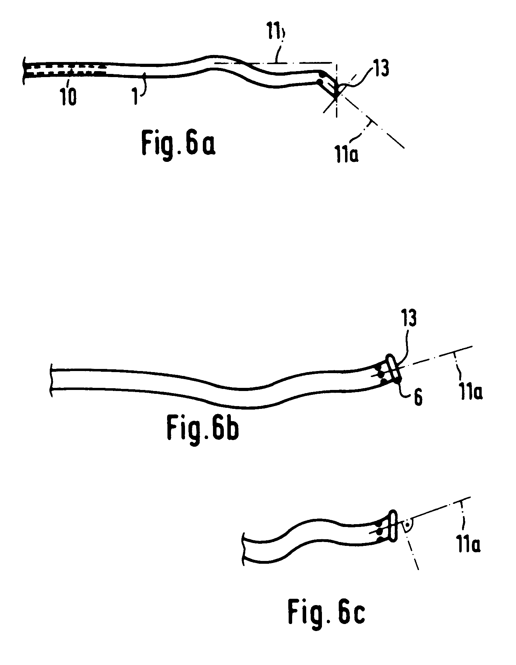

Unter Beachtung der üblicherweise vorgegebenen Außendurchmesser der Absaugkatheter sollte die maximale Amplitude A1 etwa einem mittleren Außendurchmesser des Absaugkatheters entsprechen. Die Summe, und zwar die maximale Summe A, der Wellenausschläge nach beiden Seiten der Längsachse 11 des Absaugkatheters sollte nicht über den dreifachen Außendurchmesser des Absaugkatheters hinausgehen, bevorzugt nur das Zwei- bis Zweieinhalbfache betragen. Das gewellte Ende weist insbesondere drei ausgebildete Wellenberge um die Wellenlängsachse 15, nämlich W1, W2, W3 gemäß Figur 2 auf. Das Ende des gewellten W endet in dem offenen Schenkel W4, dessen Längsachse 11a, siehe Figur 1, unter dem Winkel a gegenüber der Längsachse 11 des Absaugkatheters geneigt ist. Der Ausgang des Lumens 10 erfolgt in dem gezeigten Beispiel nach Figur 1 senkrecht zur Schenkelachse 11a. Es ist jedoch auch möglich, diesen Ausgang bzw. den Endquerschnitt so zu gestalten, daß bei abgebogenem distalem Bereich dieser dennoch senkrecht zur Längsachse 11 des Absaugkatheters sich erstreckt. Beispiele sind in den Ausführungen gemäß Figur 6a und 6c dargestellt. Hier ist der Endquerschnitt am distalen Ende 13 gegenüber der Schenkelachse 11a abgeschrägt hinterschnitten, so daß der Lumenausgang etwa senkrecht zur Längsachse 11 des Absaugkatheters verläuft und damit entsprechend axial aus der Lunge abgesaugt bzw. Medikamente in axialer Richtung 11 eingebracht werden können.

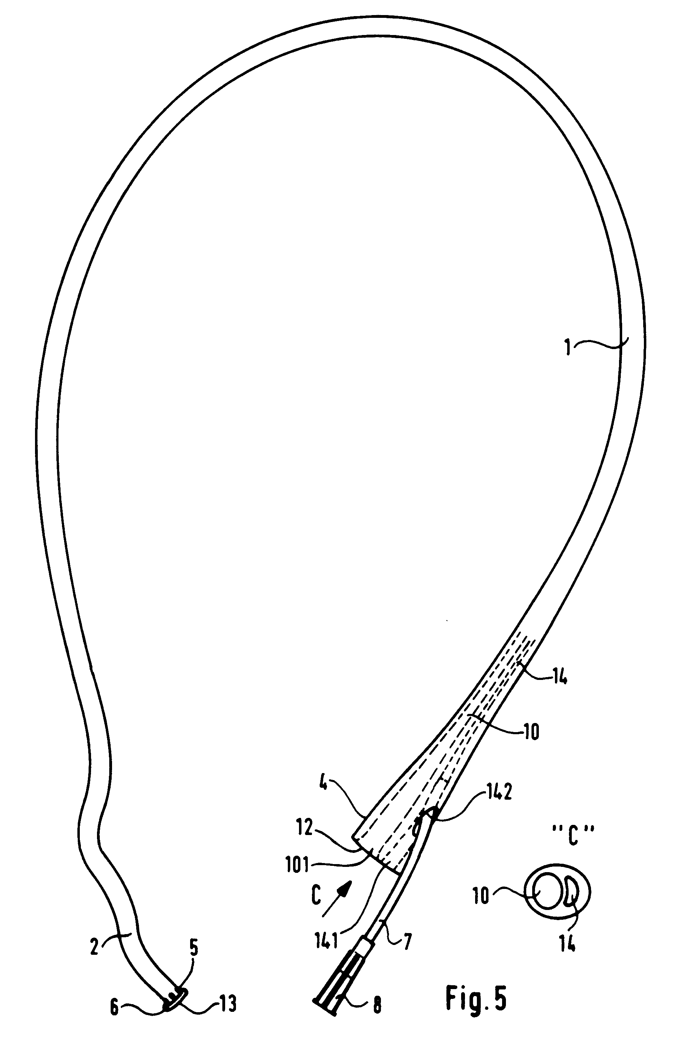

Auch bei Absaugkathetern, an deren distalen Ende eine Ringwulst 6 angeformt ist, siehe Ausschnitt Figur 6b, kann durch eine entsprechende Abschrägung des abgebogenen Schenkels, siehe Figur 6c, ein Endquerschnitt erzielt werden, der senkrecht zur Längsachse 11 des Absaugkatheters verläuft. Dies ist dann von Bedeutung, wenn zweilumige Absaugkatheter eingesetzt werden, bei denen durch ein Lumen ein Medikament möglichst tief in die Lunge eingebracht werden soll. Ein derartiger zweilumiger Absaugkatheter ist in der Figur 5 dargestellt. Er ist zusätzlich neben dem Lumen 10 zum Absaugen mit einem weiteren vom proximalen Ende bis distalen Ende durchgängigen Lumen 14 für das Durchleiten von Medikamenten ausgebildet. Das Lumen 14 weist jedoch einen kleineren Querschnitt als das Lumen 10 auf.

Das Lumen 14 hat einen Durchmesser von etwa ein bis maximal zwei Millimeter, das Lumen 10 einen Durchmesser von etwa drei bis vier Millimeter. In das Lumen 14 ist im Bereich des proximalen Endes des Absaugkatheters ein Schlauch 7 durch eine Öffnung 142 im Absaugkatheter eingesteckt, an dessen Ende ein Ansatzstück 8 für eine Spritze vorgesehen ist. Die Lumen 10, 14 sind am proximalen Ende mit ihren Eingängen 101, 141 konisch erweitert, um entsprechende Anschlüsse einstecken zu können. Am distalen Ende 13 des Absaugkatheters 1, das ebenfalls mit der Ringwulst 6 und mit radialen Öffnungen 5 ausgerüstet ist, endet das Lumen 14 in dem Lumen 10. Der distale Endbereich 2 des Absaugkatheters 1 ist in W-Form gewellt. Die Lumen 10, 14 sind nur schematisch angedeutet.

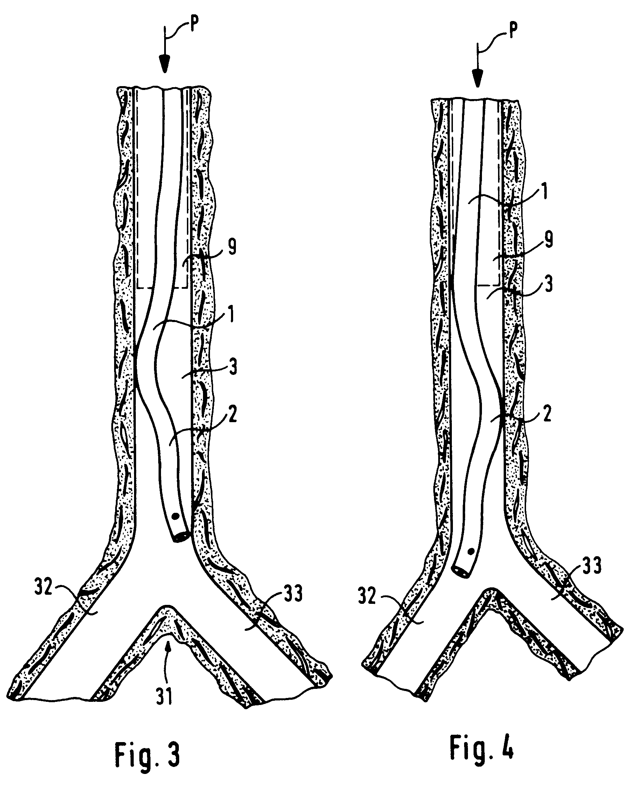

In den Figuren 3 und 4 ist die Einführung des erfindungsgemäßen Absaugkatheters 1 mit dem gewellten distalen Ende durch die Luftröhre 3 bis in die Lungenflügel 32, 33 dargestellt. Hierbei ist zweckmäßig als Einführhilfe in der oberen Luftröhre 3 noch der gestrichelt angedeutete Beatmungstubus 9 vorgesehen. Am Ende der Luftröhre gabeln sich die Gänge über der Bifurkation 31 in den rechten Lungenflügel 32 und den linken Lungenflügel 33. Je nach Führung und Haltung des Absaugkatheters 1 mit dem gewellten Endbereich 2 erfolgt beim Einschieben in Pfeilrichtung ein zwangsläufiges Einführen des Absaugkatheters gemäß Figur 3 in den linken Lungenflügel 33 über die Bifurkation oder in den rechten Lungenflügel 32 gemäß Figur 4. Die Zwangsführung des gewellten distalen Bereiches 2 des Absaugkatheters entlang der Wände, d.h. Schleimhäute, der Luftröhre und Beatmungstubus mit den Anlagepunkten ist aus den Figuren 3 und 4 erkennbar. Die Einführung des Absaugkatheters 1 erfolgt in Pfeilrichtung P. Durch einfache Drehung des Absaugkatheters 1 um seine Längsachse um 180 Grad kann derselbe Absaugkatheter abwechselnd zum sicheren Einführen in den rechten oder linken Lungenflügel benutzt werden.

Wenn der Absaugkatheter zuerst in den einen Lungenflügel eingeführt wurde, kann nach Beendigung des Absaugens der Absaugkatheter ein Stück entgegen der Pfeilrichtung P herausgezogen werden, bis er sich wieder vollständig mit dem distalen Ende innerhalb der Luftröhre 3 befindet. Danach wird der Absaugkatheter um 180 Grad gedreht und durch erneutes Nachschieben in Pfeilrichtung P in den anderen Lungenflügel eingeführt.

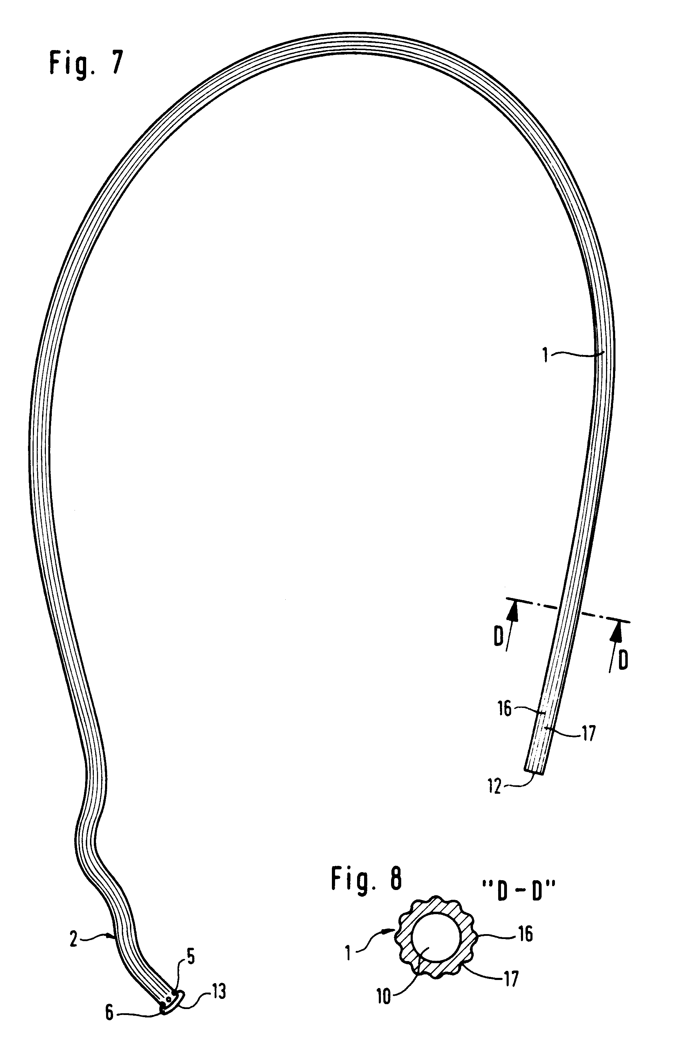

Zur Verminderung der Reibung des Absaugkatheters beim Einschieben durch den Beatmungstubus 9 bzw. die Luftröhre 3 wird der Absaugkatheter, siehe Figur 7 und 8, zweckmäßig auf seiner Oberfläche profiliert. Hierbei sind bevorzugt in Längserstreckung des Absaugkatheters verlaufende Rippen 16 und Rillen 17 ausgebildet, die gleichmäßig über den Umfang verteilt sind und sich vom proximalen Ende 12 bis zum distalen Ende 13 bzw. bis zur Ringwulst 6 oder den Löchern 5 erstrecken. Für den Fall, daß am proximalen Ende 12 des Absaugkatheters ein Anschlußstück außenseitig aufgesetzt werden soll, können in diesem Bereich die Rippen entfernt oder eingeebnet werden.

Figur 8 zeigt in Vergrößerung einen Querschnitt durch einen gerippten Absaugkatheter. Es sind viele feine Rippen gleichmäßig ausgebildet, wobei die Rippen eine Höhe bzw. die Rillen 17 eine Tiefe von etwa 30 bis etwa 700 μ aufweisen je nach Durchmesser des Absaugkatheters.

**Patentansprüche**

1. Rohrförmiger biegsamer Absaugkatheter (1) zum Einführen in die Luftröhre und das Bronchialsystem, enthaltend mindestens ein vom proximalen Ende (12) bis zum distalen Ende (13) des Absaugkatheters durchgängiges Lumen (10) zum Absaugen von Flüssigkeiten aus der Lunge, **dadurch gekennzeichnet**, daß der Bereich (2) des distalen Endes des Absaugkatheters (1) in Längserstreckung des Absaugkatheters etwa W-förmig gewellt ist und sich diese Wellung in nur einer Ebene erstreckt.

2. Absaugkatheter nach Anspruch 1, **dadurch gekennzeichnet**, daß der distale Bereich (2) auf einer Länge von etwa 40 bis 80 mm wellenförmig ausgebildet ist.

3. Absaugkatheter nach einem der Ansprüche 1 bis 2, **dadurch gekennzeichnet**, daß die Wellen des wellenförmigen Bereiches (2) zum distalen Ende (13) hin gedämpft verlaufen.

4. Absaugkatheter nach Anspruch 1, **dadurch gekennzeichnet**, daß der gewellte distale Bereich (2) sich über mindestens ein und eine halbe bis zu etwa zwei Wellenlängen erstreckt.

5. Absaugkatheter nach einem der Ansprüche 1 bis 4, **dadurch gekennzeichnet**, daß die Wellenlängsachse (15) der Wellen des distalen Bereiches (2) gegenüber der Längsachse (11) des Absaugkatheters leicht geneigt ist.

6. Absaugkatheter nach Anspruch 5, **dadurch gekennzeichnet**, daß die Wellenlängsachse (15) der Wellen des distalen Bereiches (2) um einen Winkel $\beta$ von etwa 10 bis 40 Grad gegenüber der Längsachse (11) des Absaugkatheters geneigt ist.

7. Absaugkatheter nach einem der Ansprüche 1 bis 6,

**dadurch gekennzeichnet**, daß die Größe der maximalen Amplitude (A1) des ersten Wellenberges (W1) der Wellen am distalen Bereich (2) etwa der Größe des mittleren Außendurchmessers des Absaugkatheters entspricht.

8. Absaugkatheter nach einem der Ansprüche 1 bis 7, **dadurch gekennzeichnet**, daß er einen mittleren Außendurchmesser von etwa drei bis zehn Millimeter aufweist.

9. Absaugkatheter nach einem der Ansprüche 1 bis 8, **dadurch gekennzeichnet**, daß das distale Ende (13) von einem unter einem spitzen Winkel a aus der Längsachse (11) des Absaugkatheters (1) ausgelenkten Wellenschenkel (W4) gebildet ist.

10. Absaugkatheter nach einem der Ansprüche 1 bis 9, **dadurch gekennzeichnet**, daß die Wellenausschläge des gewellten distalen Bereiches (2) aus der Längsachse (11) des Absaugkatheters (1) insgesamt ein Maß (A) nicht überschreiten, das etwa der Größe eines zwei-bis maximal dreifachen mittleren Außendurchmessers des Absaugkatheters entspricht.

11. Absaugkatheter nach einem der Ansprüche 1 bis 10, **dadurch gekennzeichnet**, daß das distale Ende (13) außenseitig mit einer Ringwulst (6) umgeben ist.

12. Absaugkatheter nach einem der Ansprüche 1 bis 11, **dadurch gekennzeichnet**, daß der Endquerschnitt des distalen Endes (13) sich etwa senkrecht zur Längsachse (11) des Absaugkatheters (1) erstreckt.

13. Absaugkatheter nach einem der Ansprüche 1 bis 4, **dadurch gekennzeichnet**, daß er außenseitig mit Längenmaßen markiert ist.

14. Absaugkatheter nach einem der Ansprüche 1 bis 3, **dadurch gekennzeichnet**, daß er mit einem in Längserstreckung des Absaugkatheters sich vom distalen bis zum proximalen Ende erstreckenden Röngtgenkontraststreifen oder dergleichen ausgerüstet ist.

15. Absaugkatheter nach einem der Ansprüche 1 bis 14,

**dadurch gekennzeichnet**, daß er außenseitig mit in Längserstreckung des Absaugkatheters (1) verlaufenden Rippen (16) ausgestattet ist.

16. Absaugkatheter nach Anspruch 15, **dadurch gekennzeichnet**, daß mindestens vier, vorzugsweise sechs oder mehr Rippen (16) einer Höhe von etwa 30 bis 700 $\mu$ gleichmäßig über den Umfang des Absaugkatheters verteilt ausgebildet sind.

## Claims

1. A flexible tubular suction catheter (1) for introduction into the trachea and the bronchial system, containing at least one lumen (10) passing from the proximal end (12) to the distal end (13) of the suction catheter for withdrawing fluids from the lung by suction, characterised in that the region (2) of the distal end of the suction catheter (1) is waved approximately in a W-shape in the longitudinal extension of the suction catheter and this waving extends in only one plane.

2. A suction catheter according to claim 1, characterised in that the distal region (2) is formed in a wave shape to a length of approximately 40 to 80 mm.

3. A suction catheter according to one of claims 1 to 2, characterised in that the waves of the wave-shaped region (2) decay towards the distal end (13).

4. A suction catheter according to claim 1, characterised in that the waved distal region (2) extends over at least one and a half to approximately two wavelengths.

5. A suction catheter according to claims 1 to 4, characterised in that the wavelength axis (15) of the waves of the distal region (2) is slightly inclined relative to the longitudinal axis (11) of the suction catheter.

6. A suction catheter according to claim 5, characterised in that the wavelength axis (15) of the waves of the distal region (2) is inclined by an angle $\beta$ of approximately 10 to 40 degrees relative to the longitudinal axis (11) of the suction catheter.

7. A suction catheter according to one of claims 1 to 6, characterised in that the size of the maximum amplitude (A1) of the first wave peak (W1) of the waves at the distal region (2) corresponds approximately to the size of the average outer diameter of the suction catheter.

8. A suction catheter according to one of claims 1 to 7, characterised in that it has an average outer diameter of approximately three to ten millimetres.

9. A suction catheter according to one of claims 1 to 8, characterised in that the distal end (13) is formed from a wave limb (W4) deflected at an acute angle a from the longitudinal axis (11) of the suction catheter (1).

10. A suction catheter according to one of claims 1 to 9, characterised in that the wave amplitudes of the waved distal region (2) from the longitudinal axis (11) of the suction catheter (1) do not exceed in total an amount (A) which corresponds approximately to the size of two to a maximum of three times the average outer diameter of the suction catheter.

11. A suction catheter according to one of claims 1 to 10, characterised in that the distal end (13) is surrounded on the outside by an annular ring (6).

12. A suction catheter according to one of claims 1 to 11, characterised in that the end cross-section of the distal end (13) extends approximately perpendicular to the longitudinal axis (11) of the suction catheter (1).

13. A suction catheter according to one of claims 1 to 4, characterised in that it is marked outside with length measurements.

14. A suction catheter according to one of claims 1 to 3, characterised in that it is provided with an x-ray contrast strip or the like extending in the longitudinal extension of the suction catheter from the distal to the proximal end.

15. A suction catheter according to one of claims 1 to 14, characterised in that it is equipped on the outside with ribs (16) extending in the longitudinal extension of the suction catheter (1).

16. A suction catheter according to claim 15, characterised in that at least four, preferably six or more, ribs (16) of a height of approximately 30 to 700 $\mu$ are distributed evenly over the periphery of the suction catheter.

## Revendications

1. Cathéter d'aspiration (1) souple tubulaire à introduire dans la trachée et le système bronchique contenant au moins une lumière (10) continue de l'extrémité proximale (12) à l'extrémité distale (13) du cathéter d'aspiration pour l'aspiration de liquides contenus dans les poumons, **caractérisé en ce** que la zone (2) de l'extrémité distale du cathéter d'aspiration (1) est ondulée en forme approximative de W dans l'axe longitudinal du cathéter d'aspiration et en ce que cette ondulation s'étend dans un seul plan.

2. Cathéter d'aspiration selon la revendication 1 **caractérisé en ce** que la zone distale (2) est ondulée sur une longueur d'environ 40 à 80 mm.

3. Cathéter d'aspiration selon une des revendications 1 à 2 **caractérisé en ce** que les ondulations de la zone ondulée (2) sont amorties vers l'extrémité distale (13).

4. Cathéter d'aspiration selon la revendication 1 **caractérisé en ce** que la zone distale ondulée (2) s'étend sur au moins une longueur d'onde et demie jusqu'à environ deux longueurs d'onde.

5. Cathéter d'aspiration selon une des revendications 1 à 4 **caractérisé en ce** que l'axe longitudinal (15) des ondes de la zone distale (2) est légèrement incliné par rapport à l'axe longitudinal (11) du cathéter d'aspiration.

6. Cathéter d'aspiration selon la revendication 5 **caractérisé en ce** que l'axe longitudinal (15) des ondes de la zone distale (2) est incliné d'un angle $\beta$ d'environ 10 à 40 degrés par rapport à l'axe longitudinal (11) du cathéter d'aspiration.

7. Cathéter d'aspiration selon une des revendications 1 à 6 **caractérisé en ce** que la valeur de l'amplitude maximale (A1) de la première crête d'onde (W1) des ondes sur la zone distale (2) correspond approximativement à la valeur du diamètre externe moyen du cathéter d'aspiration.

8. Cathéter d'aspiration selon une des revendications 1 à 7 **caractérisé en ce** qu'il présente un diamètre externe moyen d'environ trois à dix millimètres.

9. Cathéter d'aspiration selon une des revendications 1 à 8 **caractérisé en ce** que l'extrémité distale (13) est formée par un côté d'ondulation (W4) dévié de l'axe longitudinal (11) du cathéter d'aspiration (1) d'un angle aigu a.

10. Cathéter d'aspiration selon une des revendications 1 à 9 **caractérisé en ce** que les amplitudes de la zone distale ondulée (2) par rapport à l'axe longitudinal (11) du cathéter d'aspiration (1) ne dépassent pas globalement une dimension (A) qui correspond approximativement à la valeur de deux à trois fois maximum le diamètre externe moyen du cathéter d'aspiration.

11. Cathéter d'aspiration selon une des revendications 1 à 10 **caractérisé en ce** que l'extrémité distale (13) est entourée sur sa face externe d'un épaulement annulaire (6).

12. Cathéter d'aspiration selon une des revendications 1 à 11 **caractérisé en ce** que la section finale de l'extrémité distale (13) s'étend presque perpendiculairement par rapport à l'axe longitudinal (11) du cathéter d'aspiration (1).

13. Cathéter d'aspiration selon une des revendications 1 à 4 **caractérisé en ce** qu'il est marqué sur sa face externe par des mesures de longueur.

14. Cathéter d'aspiration selon une des revendications 1 à 3 **caractérisé en ce** qu'il est muni d'une bande de contraste aux rayons X ou autre s'étendant dans la direction longitudinale du cathéter d'aspiration de l'extrémité distale jusqu'à l'extrémité proximale.

15. Cathéter d'aspiration selon une des revendications 1 à 14 **caractérisé en ce** qu'il est pourvu sur sa face externe de nervures (16) s'étendant dans la direction longitudinale du cathéter d'aspiration (1).

16. Cathéter d'aspiration selon la revendication 15 **caractérisé en ce** qu'au moins quatre nervures (16), de préférence six ou plus, d'une hauteur d'environ 30 à 700 $\mu$ sont réparties uniformément sur la périphérie du cathéter d'aspiration.

# Fig. 1

# Fig. 2

Fig. 3

Fig. 4

Fig.5

Fig. 6a

Fig. 6b

Fig. 6c

# Fig. 7

# Fig. 8   "D - D"